# EUROPEAN PATENT APPLICATION

(11) **EP 1 040 802 A1**
(43) Date of publication of application: **04.10.2000**
(21) Application number: 99105194.7
(22) Date of filing: 01.04.1999
(51) Int. Cl.: A61F 13/15

(54) **Absorbent article with slanted capillaries apertured backsheet**

(71) Applicant: THE PROCTER & GAMBLE COMPANY, Cincinnati, Ohio 45202 (US)
(72) Inventor: Marinelli, Luigi, 65129 Pescara (IT); D'Incecco, Amedeo Franco, 65125 Pescara (IT); Veglio, Paolo, 65125 Pescara (IT); Carlucci, Giovanni, 66100 Chieti (IT); Cimini, Carmine, 65812 Bad Soden (DE)
(74) Representative: Hirsch, Uwe Thomas M.H.

(57) **Abstract**

The present invention relates to absorbent articles like baby diapers, adult incontinence articles and in particular to sanitary napkins or pantiliners which have an apertured film topsheet on the surface facing the wearer in use. According to the present invention the articles are provided also with an apertured backsheet for breathability. The backsheet comprises at least one breathable layer of a resilient, three dimensional web which consists of a liquid impervious polymeric film having apertures. These apertures form capillaries which are not perpendicular to the plane of the film but are disposed at an angle of less than 90° relative to the plane of the film. The capillaries also have a length L which has a preferred value as compared to the distance that separates a capillary from adjacent ones.

## Description

### FIELD OF THE INVENTION

The present invention relates to absorbent articles like baby diapers, adult incontinence articles and in particular to sanitary napkins or pantiliners. According to the present invention the articles are provided also with an apertured backsheet for breathability. The backsheet comprises at least one breathable layer of a resilient, three dimensional web which consists of a liquid impervious polymeric film having apertures. These apertures form capillaries which are not perpendicular to the plane of the film but are disposed at an angle of less than 90° relative to the plane of the film. The capillaries also have a length L which has a preferred value as compared to the distance that separates a capillary from adjacent ones.

### BACKGROUND OF THE INVENTION

The primary consumer needs which underlie development in the absorbent article field, in particular sanitary napkins, catamenials, or pantiliners is the provision of products providing both a high protection and comfort level.

One means for providing consumer comfort benefits in absorbent articles is by the provision of breathable products. Breathability has typically concentrated on the incorporation of so called 'breathable backsheets' in the absorbent articles. Commonly utilised breathable backsheets are microporous films and apertured formed films having directional fluid transfer as disclosed in for example US 4591523. Both these types of breathable backsheets are vapour permeable allowing gaseous exchange with the environment. This thereby allows for the evaporation of a portion of the fluid stored in the core and increases the circulation of air within the absorbent article. The latter is particularly beneficial as it reduces the sticky and soiled feeling experienced by wearers of or associated with articles comprising an apertured formed film or film like topsheet.

The use of apertured film topsheets has long been recognized as providing particular benefits in controlling the liquid flow through this layer into an absorbent structure and reducing the liquid flow out of the absorbent structure towards the skin of a wearer. In this respect apertured film topsheets have provided an exceptional dryness comfort to the wearers of absorbent articles, particularly sanitary napkins. This comfort benefit, however, started to wear off under stress conditions of such articles, such as physical exercising of the wearer (which also caused transpiration from the skin opposite the article to emanate more strongly), heavy loading of the article or extended wearing duration.

A drawback associated with the use of breathable backsheets in absorbent articles is the negative effect on the protection level performance by leakage, known as wet through, onto the users garment. Although, breathable backsheets in principle only allow the transfer of materials in the gaseous state, physical mechanisms such as extrusion, diffusion and capillary action may still occur and result in the transfer of the fluids from the absorbent core through the backsheet and onto the user's garments. In particular, these mechanisms become more dominant if the product is utilised during physical exertion, or for heavy discharge loads or over extended periods of time. Thus, whilst the incorporation of breathable backsheets in absorbent articles is highly desirable from a comfort standpoint, since the primary role of a backsheet still remains the prevention of liquid leakage, conventional breathable backsheets have not been successfully incorporated into products.

The problem of wet through onto users garments due to the incorporation of such breathable backsheets in absorbent articles has indeed also been recognized in the art. Attempts to solve the problem have mainly resided in the use of multiple layer backsheets such as those illustrated in US 4341216. Similarly European patent application no. 710471 discloses a breathable backsheet comprising an outer layer of a gas permeable, hydrophobic, polymeric fibrous fabric and an inner layer comprising an apertured formed film having directional fluid transport. The backsheet construction preferably has no liquid transport/wet through under certain specified test conditions. Also European patent application no. 710472 discloses a breathable backsheet consisting of at least two breathable layers which are unattached to one another over the core area. The backsheet construction preferably has no liquid transport/wet through under certain specified test conditions.

US 4713068 discloses a breathable clothlike barrier for use as an outer cover for absorbent articles. The barrier comprises at least 2 layers, a first layer having a specified basis weight, fiber diameter and pore size and a second layer comprising a continuous film of poly (vinyl alcohol) having a specified thickness. The barrier also has a specified water vapour transmission rate and level of impermeability.

However, these proposed solutions have not been able to provide a fully satisfactory solution to the problem of breathable backsheet wet through under stress conditions. But especially under such stress conditions breathability would have most pronounced comfort benefits especially for articles comprising film topsheets. For such articles the experienced or assumed stickiness, stuffiness, or soil residue between film and skin is greatest under stress conditions.

US 5591510 as well as WO 97/03818 and WO 97/03795 disclose an apertured film layer having capillaries which are disposed at an angle relative to the plain of the film, which films are referred to as slanted capillary films. This film structure is provided as an improvement for incorporation into clothing and garments which are breathable, yet non transmitting liquids toward the wearer of such garments. Also the use of such slanted capillary films is indicated in the context of absorbent articles but as a topsheet, particularly in figure 16 of US 5591510 the combination of such slanted capillary films together with an absorbent material is disclosed, however not in the context of disposable absorbent articles according to the present invention.

In European patent applications Nos. 98101867.4 and 98101868.2, respectively entitled "Absorbent article with breathable dual layer backsheet comprising one layer with slanted capillary apertures", and "Absorbent article with breathable backsheet comprising slanted capillary apertures and an apertured film topsheet", disposable absorbent articles are described such as baby diapers, adult incontinence articles and in particular sanitary napkins or panty liners. Typically such articles comprise a liquid pervious topsheet forming the wearer facing surface of the article, an absorbent core and a breathable backsheet forming the garment facing surface of the article. The absorbent core is interposed between the topsheet and the backsheet.

The breathable backsheet is located on the garment facing surface of the absorbent core and comprises at least one backsheet layer. The backsheet comprises a resilient three dimensional web, which consists of a liquid impervious polymeric backsheet film which backsheet film has apertures. The apertures form capillaries which have side walls which extend away from the wearer facing surface of the backsheet film and towards the absorbent core providing the web with three dimensionality. The capillaries have a first opening in the garment facing surface of the backsheet film and a second opening at the end of the capillaries spaced apart from the wearer facing surface of the backsheet film. The capillaries are slanted, i.e., they extend away from the wearer facing surface of the backsheet film at an angle which is less than 90° with respect to the plane of the backsheet film, or, in other embodiments, are curved or bent towards the plane of the backsheet film. Further, in alternative or in addition thereto, the capillaries can have a first and a second portion which are different in direction, form, shape, size or combinations thereof.

Also the second opening of at least some of the capillaries may be provided as slits. Slits are considered to be such forms in which the longest extent of an opening is at least 5 times the length of the smallest length of the opening.

Owing to the directional liquid transport capability of the polymeric backsheet film and to the ability to close under pressure derivable from the angled (slanted) capillaries the breathable backsheets of the above mentioned European patent applications provide a sanitary article with a good leak through protection while maintaining optimum breathability for improved comfort.

However, the performances of breathable backsheets comprising at least a resilient three dimensional polymeric film having apertures that form slanted capillaries as described above, can still be improved in terms of a better resistance to wet through under the pressure and stress experienced during the use of the absorbent articles comprising said backsheets, combined with a good breathability.

The ability to bend under pressure of the slanted capillaries in the preferred resilient three dimensional polymeric films, incorporated in breathable backsheets as mentioned above, on one hand in fact provides said films with the capacity to close the apertures under pressure, therefore giving them a substantial liquid imperviousness under compression, when leakage is more likely to occur due to fluid that can be e.g. squeezed from the absorbent core through the breathable backsheet. At the same time, however, and in particularly severe wearing conditions that can result in higher compression forces exerted onto the three dimensional apertured polymeric film, typically in a direction perpendicular to the film plane, the bending of the slanted capillaries can be so pronounced that it can bring the second opening at the end of the capillaries in direct contact with the inner surface of the three dimensional polymeric film which lies between the capillaries themselves, where a certain amount of fluid can accumulate during the use of the article, e.g. coming form the absorbent core. This small amount of fluid does not cause any inconvenience during normal use conditions, since it is located and confined in the "valleys" among the capillaries on the surface of the three dimensional polymeric film facing towards the absorbent core. But under particularly heavy conditions of compression and stress some of this fluid can be sucked by capillary action into the second opening of the capillaries, according to the above explained mechanism, and when the pressure is released, and the capillaries recover their original, open configuration, this fluid could cause wet through by leaking from the opposite, garment facing surface of the three dimensional polymeric film.

It is therefore an object of the present invention to provide a breathable disposable absorbent article having a breathable backsheet comprising an improved resilient three dimensional polymeric film with apertures that form capillaries that are angled (slanted) with respect to the plane of the three dimensional film, wherein said breathable backsheet is also exceptionally resistant to wet through under the stresses and compressions experienced during the use.

Such an increased resistance is even more desirable when the resilient three dimensional polymeric film comprises slanted capillaries which are preferably particularly slender, i.e. being provided with a higher length as compared to the dimension of the respective first opening in the garment facing surface of the polymeric backsheet film, in order to facilitate bending, as descried in the European patent application filed by the applicant at the same time as the present application, and entitled "Absorbent article with improved backsheet comprising capillary apertures" (P&G Case CM2062F).

### SUMMARY OF THE INVENTION

The present invention relates to breathable disposable absorbent articles of a layered construction for absorption of body fluids, such as baby diapers, adult incontinence articles and in particular sanitary napkins or panty liners. Also articles such as underarm sweat pads or scholar shirts may benefit from the present invention. Typically such articles are of layered construction with each layer or group of layers having a garment facing surface which is oriented to face in the direction of a garment during use of the article and a wearer facing surface facing in the opposite direction. Typically such articles comprise a liquid pervious topsheet forming the wearer facing surface of the article, an absorbent core and a breathable backsheet forming the garment facing surface of the article. The absorbent core is interposed between the topsheet and the backsheet.

The breathable backsheet is located on the garment facing surface of the absorbent core and comprises at least one backsheet layer. The backsheet comprises at least a resilient three dimensional web, which consists of a liquid impervious polymeric backsheet film having a garment facing surface and a wearer facing surface, both surfaces being planar and parallel to each other, and parallel to the plane P of the backsheet film. The backsheet film has apertures. The apertures form capillaries which have side walls which extend away from the wearer facing surface of the backsheet film towards the absorbent core providing the web with three dimensionality. The capillaries have a first opening in the garment facing surface of the backsheet film and a second opening at the end of the capillaries spaced apart from the wearer facing surface of the backsheet film.

Each of the capillaries defines a conduit constituted by the first opening, the second opening and the side walls. Each of the capillaries also has a centreline defined as the geometrical axis of the conduit, and a length measured along the centreline between two points P₁ and P₂ respectively corresponding to the first opening and the second opening as described herein.

The centreline A forms along at least part of its length L an angle of less than 90 degrees measured from the plane P, with the centreline A comprised in a plane P' perpendicular to the plane P.

Each one of the capillaries is separated from adjacent capillaries by distances measured along the garment facing surface of the backsheet film between respective first openings of the capillary, and of the adjacent capillaries, wherein the distance measured along the line of intersection of the plane P with the plane P' is B. The first opening has also a dimension D measured along the direction of the distance B and passing through the point P₁, wherein the length L is longer than the sum of the distance B plus half of the dimension D.

### BRIEF DESCRIPTION OF THE DRAWINGS

Figure 1 shows a cross-sectional view of an absorbent article comprising all usual elements of such articles including an embodiment of the breathable backsheet according to the present invention.

Figure 2 shows a plan view of the garment facing surface of a portion of the three dimensional polymeric film used in the breathable backsheet of the present invention comprised in the absorbent article of Figure 1.

Figures 3 - 7 show particular alternative embodiments of the slanted capillaries used for the three dimensional web comprised in the breathable backsheet according to the present invention.

### DETAILED DESCRIPTION OF THE INVENTION

The present invention relates to disposable absorbent articles for absorption of body fluids, such as sanitary napkins, panty liners, incontinence products sweat pads and baby diapers. Typically such articles comprise the elements of a liquid pervious topsheet, a backsheet and an absorbent core intermediate said topsheet and said backsheet. According to the present invention the topsheet, backsheet and core may be selected from any of the known types of these components provided that they meet the desired comfort and protection performance requirements and conditions noted below and in the appended claims.

In general, the topsheet - if present - should have such a liquid retention to maintain a dry surface and thereby keep the skin of the wearer dry; the absorbent core needs to provide enough absorbent capacity and allow the flow of vapour and/or air through it and the backsheet should prevent wet through (liquid permeability) to retain the absorbed fluid while being sufficiently breathable. Furthermore, the individual elements are joined, preferably using techniques such that the final product has the desired comfort and performance level.

In the following description of the invention the surface facing in the direction of the wearer is called wearer facing surface. In the drawings this direction is indicated by arrow 20. Further the surface facing in the direction of the garment is called garment facing surface and in the drawings this direction is indicated by arrow 21.

### Absorbent article components

### The topsheet

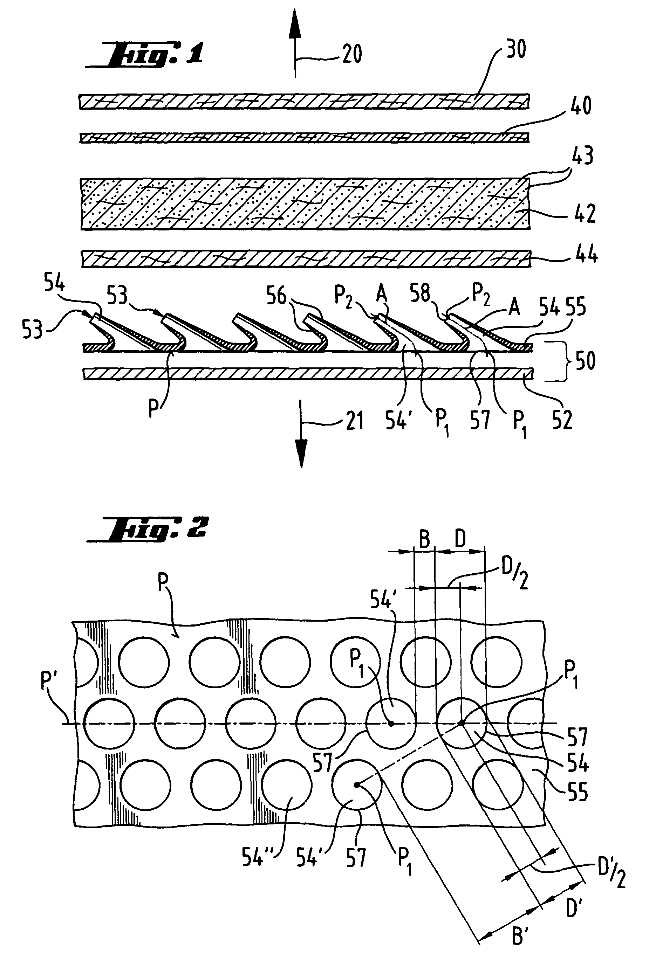

According to the present invention the absorbent article usually comprises a topsheet. In a disposable absorbent article the topsheet generically comprises the topmost layer or layers intended to directly contact the wearer's body. The topsheets suitable for use herein may be any topsheet known in the art. In Figure1 the topsheet is indicated with reference numeral 30.

The topsheets for use herein may comprise a single layer or a multiplicity of layers. In a preferred embodiment the topsheet comprises a first layer which provides the user facing surface of the topsheet and a second layer between the first layer and the absorbent structure/core. In addition another layer on the wearer facing surface of the first layer but only extending in the central zone or in parts of the peripheral zone of the article can be desirable to provide extra softness or extra liquid handling/retaining abilities (this design is usually referred to as "hybrid topsheet"). The topsheet typically extends across the whole of the absorbent structure and can extend into and form part of or all of the preferred side flaps, side wrapping elements or wings. Also the topsheet (or rather at least one layer thereof) can wrap around the absorbent core, thereby providing a topsheet layer and a layer which is considered part of the backsheet.

The topsheet as a whole and hence each layer individually needs to be compliant, soft feeling, and non-irritating to the wearer's skin. It also can have elastic characteristics allowing it to be stretched in one or two directions. As used herein the topsheet hence refers to any layer or combination of layers whose principle function is the acquisition and transport of fluid from the wearer towards the absorbent core and containment of the absorbent core. In addition the topsheet of the present invention should have a high vapour permeability, preferably also a high air permeability.

According to the present invention the topsheet may be formed from any of the materials available for this purpose and known in the art, such as wovens, nonwovens, films or combinations thereof. In a preferred embodiment of the present invention at least one of the layers of the topsheet comprises a liquid permeable apertured polymeric film. One layer, but preferably the wearer facing and contacting layer, is provided by a film material having apertures which are provided to facilitate liquid transport from the wearer facing surface towards the absorbent structure. Such liquid permeable, apertured films are well-known in the art. They provide a resilient three dimensional fiber-like structure. Such films have been disclosed in detail for example in US 3929135, US 4151240, US 4319868, US 4324246, US 4342314, US 4591523, US 4609518, US 4629643, US 4695422 or WO 96/00548.

An example of such film is available from the Procter & Gamble Company, Cincinnati, Ohio, USA under the trade name Dryweave. Also such films are available from the Company Pantex from Pistoia, Italy under the designation "PF-films". Also film according to US 5591510 or WO 97/03818 and WO 97/03795 described for use as a layer in breathable backsheets can be employed but may require modification of the apertures to ensure liquid permeability from the wearer facing surface to the absorbent core which is the primary objective of the topsheet and the layers constituting it. Such modification can e.g. be a surface energy alteration which actively drives liquids into and through apertures by creating a gradient of surface tension of the film. A method to provide surface energy gradients is disclosed e.g. in WO 96/00548.

A particularly preferred design would then be to use the same film for the topsheet and the backsheet both possibly supplemented by additional layers. Such a film is e.g. wrapped around and encircles the absorbent core and is treated for liquid transport into the absorbent core in the area corresponding to the topsheet but is not treated in the area corresponding to the longitudinal sides and to the backsheet (or treated to prevent liquid migration from the absorbent core through the backsheet). Treatment in the area corresponding to the topsheet can e.g. provide a discontinuous coating of hydrophobic silicone on the wearer facing surface in line with WO 96/00548. In addition or alternatively the characteristics of the apertures can be made differently (i.e. for liquid transport) in the film area corresponding to the topsheet.

### Absorbent core

According to the present invention the absorbent cores suitable for use herein may be selected from any of the absorbent cores or core system known in the art. As used herein the term absorbent core refers to any material or multiple material layers whose primary function is to absorb, store and distribute fluid. In Figure 1 the absorbent structure is shown to comprise 3 layers 40, 42, and 44.

The absorbent core of the present invention should have a high vapour permeability preferably also a high air permeability. The absorbent core preferably has a caliper or thickness of less than 12 mm, preferably less than 8 mm, more preferably less than 5 mm, most preferably from 4 mm to 2 mm.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### Primary/Secondary Fluid Distribution Layer

One optional component of the absorbent core according to the present invention, indicated as layer 40 in Figure 1, is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers.

### b Fluid Storage Layer

Positioned in fluid communication with, and typically underlying the primary or secondary distribution layers, is a fluid storage layer 42. The fluid storage layer can comprise any usual absorbent material or combinations thereof. It preferably comprises absorbent gelling materials usually referred to as "hydrogel", "superabsorbent", hydrocolloid" materials in combination with suitable carriers, which are indicated as particles 43 in Figure 1.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for,use herein will most often comprise particles of a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate. These layers can be joined to each other for example by adhesive or melting a polymeric powder binder (e.g. PE powder), by mechanical interlocking, or by hydrogen bridge bends. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

An alternative are foam like or actual foam structures as liquid storage. There are open cell foams which absorb liquid and through chemical or surface interaction retain the liquid also under pressure. Typical foams in this context are e. g. those disclosed in PCT publications WO 93/03699, WO 93/04092, WO 93/04113.

### c Optional Fibrous ("Dusting") Layer

An optional component for inclusion in the absorbent core according to the present invention is a fibrous layer adjacent to, and typically underlying the storage layer identified by reference numeral 44 in Figure 1. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention, and preferably is provided close to or as part of the primary or secondary fluid distribution layer or the fluid storage layer, are odor control agents such as zeolites, carbon black, silicates, EDTA or other chelates. Such agents are preferably provided in particulate form or as part of particles and can be provided together with the absorbent gelling material mentioned supra.

### Backsheet

The absorbent article according to the present invention also comprises a breathable backsheet. The backsheet primarily has to prevent the exudates absorbed and contained in the absorbent structure from wetting articles that contact the absorbent product such as underpants, pants, pyjamas, undergarments, and shirts or jackets, thereby acting as a barrier to fluid transport. In addition however, the breathable backsheet of the present invention permits the transfer of at least water vapour, preferably both water vapour and air through it and thus allows the circulation of air into and water vapour out of the article. The backsheet typically extends across the whole of the absorbent structure and can extend into and form part or all of side flaps, side wrapping elements or wings, if present.

According to the present invention suitable breathable backsheets for use herein comprise at least one liquid impervious polymeric backsheet layer. The backsheet comprises a resilient three dimensional web which consists of a liquid impervious film which has apertures forming slanted capillaries and is air permeable. Preferred breathable backsheets for use herein are those having a high vapour exchange, most preferably both a high vapour and high air exchange. The film is oriented such that it retards or prevents liquid from passing from the absorbent core towards the outside while allowing free air flow through it.

According to the present invention any additional backsheet layer needs to provide at least water vapour permeability so as to support breathability of the article. It is not required but desirable that it also supports air permeability in order to further improve the comfort benefit from the breathability of the article. In this context suitable water vapour and air permeable layers include two-dimensional micro- or macro-apertured films, which can also be micro- or macroscopically expanded films, formed apertured films and monolithic films, as well as nonwovens, or wovens. Such films are disclosed in detail e.g. in EP 293482 and the references therein, or US 3929135, US 4637819 and US 4591523.

Suitable two dimensional planar layers that can be comprised in the backsheet as additional layers may be made of any material known in the art, but are preferably manufactured from commonly available polymeric materials. Suitable materials are for example Goretex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures do not protrude out of the plane of the layer. The apertured materials for use as a backsheet in the present invention may be produced using any of the methods known in the art such as described in EP 293482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3929135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films have a directional liquid transport and are positioned such that they support the prevention of liquid loss (leakage) through the backsheet. Suitable macroscopically expanded films for use herein include films as described for example in US 4637819 and US 4591523.

Suitable monolithic films include Hytrel (TM), available from DuPont Corporation, USA, and other such materials as described in Index 93 Congress, Session 7A "Adding value to Nonwovens", J-C. Cardinal and Y. Trouilhet, DuPont de Nemours international S.A, Switzerland. Suitable non-wovens and/or wovens are any of those well known in the art. Non-wovens such as spunbonded, melt blown or carded which are thermobonded airlaid, drylaid or even wetlaid with or without binder can be used. Particularly preferred non-wovens are multilayer non-wovens such as a composite of fine melt blown fibers with more coarse spunbonded fibers with the meltblown fibers forming the wearer facing surface of the non-woven layer.

The resilient three dimensional liquid impervious film comprised in the backsheet according to the present invention provides air and water vapour permeability by being apertured, with apertures forming slanted capillaries. In a preferred embodiment of the present invention illustrated in figure 1 the breathable disposable absorbent article, which can be for example a sanitary napkin or a pantiliner, comprises a breathable backsheet 50. The breathable backsheet 50 comprises a resilient, three dimensional liquid impervious polymeric backsheet film 55 having a garment facing surface and a wearer facing surface being planar and parallel to each other, and also parallel to the plane P of the backsheet film 55. In the embodiment of the present invention shown in Figure 1, the plane P actually corresponds to the garment facing surface of the backsheet film 55.

The terms "planar" and "plane" as used herein refer to the configuration of the backsheet film 55 as such, as if the backsheet film alone were laid on a flat surface, even if the backsheet film 55 may also be not planar when comprised in an e.g. non planar absorbent article according to an alternative embodiment of the present invention.

The backsheet film 55 also comprises apertures 53 which form slanted capillaries 54, and a second layer 52 which is also breathable, as an additional backsheet layer as described above.

The breathable backsheet 50 according to an alternative embodiment of the present invention can however be constituted only by a resilient three dimensional web 55 as described above, with no additional backsheet layer.

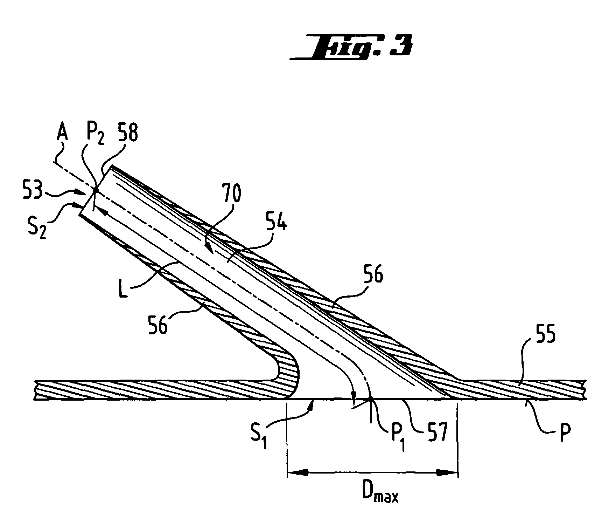

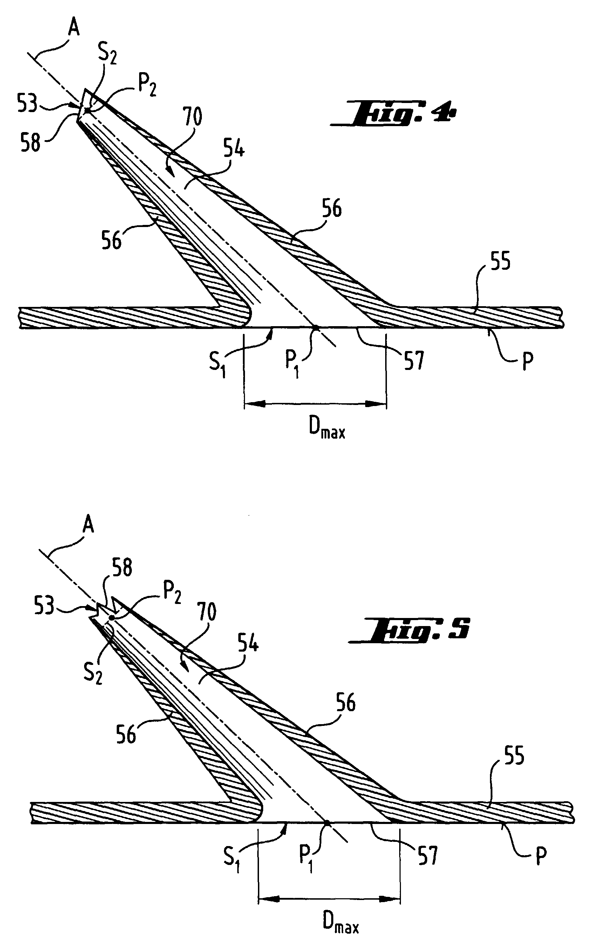

Figure 3 shows a cross-sectional view of a single slanted capillary 54 very similar to those comprised in the polymeric backsheet film 55 of Figure 1, but further featuring a preferred ratio between its length and the dimension of its first opening in the garment facing surface of the film 55 (plane P), as described in the European patent application filed by the applicant at the same time as the present application, and entitled "Absorbent article with improved backsheet comprising capillary apertures" (P&G Case CM2062F). The slanted capillary 54 have side walls 56 which extend away from the wearer facing surface of the polymeric backsheet film 55 (plane P) towards the absorbent core 40, 42, 44. The capillaries 54 have a first opening 57 in the plane of the garment facing surface of the polymeric backsheet film 55 (corresponding to the plane P of the backsheet film 55), and a second opening 58 at the end of the capillaries 54 which is spaced apart from the wearer facing surface of the polymeric backsheet film 55. Each capillary 54 defines a conduit 70 constituted by the first opening 57, the second opening 58, and the side walls 56. Each capillary 54 also has a centreline A defined as the geometrical axis of the conduit 70. In the embodiment of Figure 3, where the conduit 70 has a cylindrical shape, the centreline A corresponds to the straight line constituting the axis of the cylinder at least in the portion of the capillary 54 where the conduit 70 is entirely defined as a cylinder. In the region where the cylindrical conduit 70 merges the plane P of the backsheet film 55 the centreline A is curved as shown in Figure 3. In alternative embodiments of the present invention, where the conduit 70 defined by the capillaries 54 can have different shapes, such as for example those illustrated in figures 3-8, the centreline A is in fact not necessarily a straight line, but can be a curved line corresponding to the geometrical axis of the conduit, identified as known from solid geometry.

In most preferred embodiments of the present invention as those illustrated in the attached drawings, where the sectional views of Figures 3 to 7 are taken from a symmetry plane perpendicular to the plane P of the backsheet film, the identification of the centreline A of the conduit 70 can be more simply done on these same sectional views, since said centreline A entirely lies on this symmetry plane. For example, in the region where the conduit 70 merges the plane P of the backsheet film 55 in Figures 3-7 the identification of the centreline A as the geometrical axis of the conduit 70 must take into account the fact that the conduit 70 typically has not a regular shape in this region, as compared to the remaining portion of the capillary 54 where the conduit 70 is entirely defined as a cylinder or a truncated cone, and where the centreline A is straightforwardly defined. A good approximation to identify the centreline A in this merging region is to connect the points at the centre of some sections of the conduit 70 which are parallel to the plane P, and are taken at different distances from the plane P (which surfaces of course correspond to segments parallel to the plane P in the section views of the drawings). This practical method can be used to draw the centrelines in the conduits 70 illustrated in Figures 3 to 7. This is also consistent with the identification of the intersection point between the centreline A and the surface of the first opening 57 in the plane P of the backsheet film 55, as will be explained hereinafter.

The first opening 57 of the capillary 54 in the garment facing surface of the polymeric backsheet film 55 has a smallest dimension Dₘᵢₙ and a largest dimension Dₘₐₓ respectively defined as the minimum and the maximum open measurements within the area of the first opening 57 in the plane of the garment facing surface of the polymeric backsheet film 55 (plane P), i.e., being measured when said garment facing surface of the polymeric backsheet film 55 is laying on a flat surface. The smallest and the largest dimensions Dₘᵢₙ and Dₘₐₓ can be evaluated with means known in the art, such as for example by image analysis techniques.

An average dimension Dₐᵥ can also be evaluated for the first opening 57, as corresponding to the hydraulic diameter of a conduit having the same cross-section as the first opening 57 in the plane of the garment facing surface of the polymeric backsheet film 55, wherein the whole perimeter of said first opening 57 is wetted by the fluid. Of course the concept of "hydraulic diameter" is used herein by way of definition only, since the conduit 70 of the capillaries 54 is not intended for transport of liquids, and the term "fluid" is therefore used in its broader meaning comprising gaseous fluids, namely air and/or water vapour. As it is well known from hydraulics, the hydraulic diameter of a conduit having a cross-sectional area and a wetted perimeter corresponds to four times the ratio between said cross-sectional area and said wetted perimeter, that in turn corresponds in this context to the entire perimeter of the cross-sectional area of the first opening 57.

In the embodiment illustrated in figure 3, where the conduit 70 has the shape of a cylinder having a certain diameter, which extends away from the wearer facing surface of the film 55 at an angle of less than 90 degrees, the first opening 57 in the garment facing surface of the polymeric backsheet film 55 has typically an elliptic shape with the two axes larger than respective axes of an ellipse simply formed by the intersection of the same cylinder with the plane P at the same angle, owing to the curvature imparted to the film 55 around the first opening by e.g. the manufacturing method and apparatus used for the formation of the polymeric backsheet film 55.

In this case it is this larger major axis that corresponds to the largest dimension Dₘₐₓ of the first opening 57, while typically the larger minor axis will correspond to the smallest dimension Dₘᵢₙ.

Each capillary 54 also has a length L measured along the centreline A between the first opening 57 and the second opening 58. The length L is intended to be measured along the centreline A between two points of intersection P₁ and P₂ defined as follows.

The first point P₁ corresponds to the intersection of said centreline A with the surface S₁ of the first opening 57. Said surface S₁ is flat, as can be seen in the embodiments illustrated in the drawings, and, belongs to the plane of the garment facing surface of the polymeric backsheet film 55 (plane P). From a practical point of view, in the embodiments of the present invention such as those illustrated in Figures 3 to 7, the first point P₁ corresponds to the centre of the first surface S₁, as already explained with reference to the identification of the centreline A of the conduit 70 of the capillaries 54 having different shapes.

The second point P₂ corresponds to the intersection between the centreline A of the conduit 70 and a flat surface S₂ which is perpendicular to said centreline and passes through the point or points of the perimeter of the second opening 58 which is/are closer to the respective first opening 57 of the capillary 54, wherein said distance is evaluated along the inner surface of the side wall 56 of the capillary 54.

Also these measurements can be obtained with methods known in the art, for example by means of image analysis performed on photomicrographs of the polymeric backsheet film 55 taken with a scanning electron microscope.

In the embodiment of Figure 3, where the capillary 54 has a regular cylindrical shape along most of its length, and the second opening 58 also has a regular circular perimeter, the flat surface S₂ perpendicular to the centreline A which identifies the intersection point P₂ corresponds to a flat circular surface passing through the whole perimeter of the second opening 58. It is generally preferred that said second opening 58 be as smooth as possible so as not to create a liquid transport entanglement between the extending elements at the end of the second opening 58 of the capillary 54 with the absorbent core 44 in the absorbent article (in contrast this may be desirable for use of such a film as an apertured film topsheet where such loose elements provide the function of sucker feet to enhance liquid transport). However, in other cases, and depending on the method used for the formation of the polymeric backsheet film 55, the perimeter and shape of the second aperture 58 may not be regular.

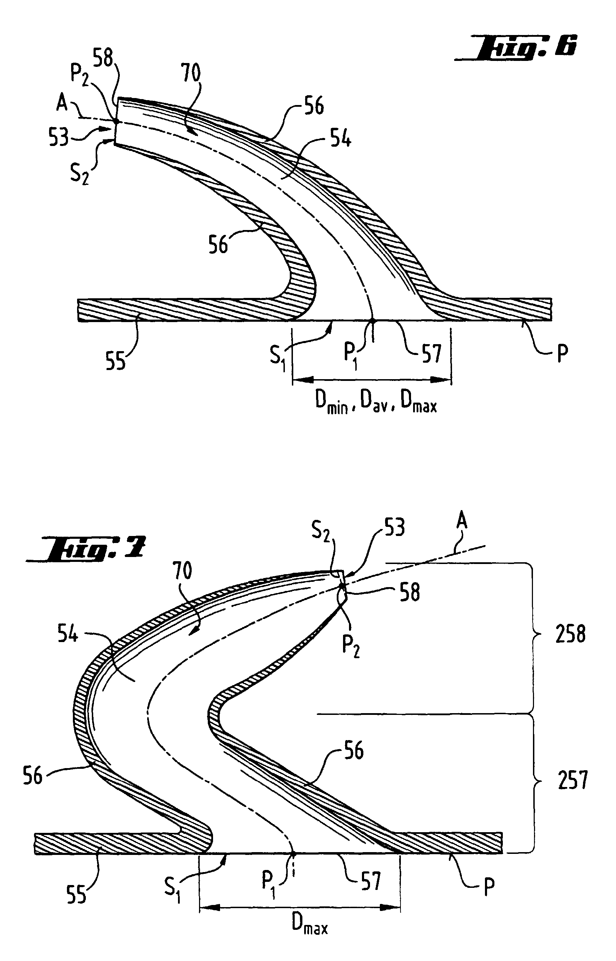

For example, the edge of the second opening 58 may be rugged or uneven, comprising loose elements extending from the edge of the opening, as illustrated in Figure 5. In this embodiment, the surface S₁ of course lies in the plane of the garment facing surface of the polymeric backsheet film 55, such as the corresponding surface S₁ of figure 2, while the surface S₂, defined as explained above with reference to the identification of the second point P₂, is indicated as a section in Figure 5 with a dotted line.

In Figures 4 to 7 are shown by way of example the centrelines A and the corresponding features S₁, S₂, P₁, P₂, in capillaries 54 having different and less regular shapes. Usually the centreline A passes through a first point P₁ which corresponds to the centre of the surface S₁, identified for regular and irregular shapes of the surface S₁ as it is known from plane geometry. In the sectional view of Figure 6 the dimensions Dₘᵢₙ, Dₐᵥ, and Dₘₐₓ of the first openings 57 coincide, since it is considered that the first opening 57 has a circular shape in said figure.

On the contrary, in Figures 4, 5, and 7, the surfaces S₁ has typically an elliptical shape, with the longer axis shown in the sectional views. In these cases the dimension shown in these figures corresponds to the largest dimension Dₘₐₓ as defined herein.

For each capillary 54 of the polymeric backsheet film 55 comprised in the breathable absorbent article of the present invention a ratio can be defined between the length L of the capillary 54, and the dimension D of the respective first opening 57, wherein D can correspond to Dₘᵢₙ, Dₐᵥ, or Dₘₐₓ.

According to a particularly preferred embodiment of the present invention, by selecting this ratio it is possible to improve the performances of a disposable absorbent article with a breathable backsheet incorporating the polymeric backsheet film 55 with slanted capillaries 54, in terms of increased breathability combined with a better wet through resistance of the structure of the breathable backsheet under the pressure and stress typically exerted by the user on such a disposable absorbent article, for example a sanitary napkin or pantiliner, during the wearing time.

Resiliency and cushioning effect in response to compressive forces exerted substantially perpendicularly to the polymeric backsheet film surface can also be improved according to this preferred embodiment.

Accordingly, the ratio between the length L of the slanted capillaries 54 and the smallest dimension Dₘᵢₙ of the first opening 57 is preferably greater than 2. More preferably, the ratio between the length L and the average dimension Dₐᵥ of the first aperture 57 should be greater than 2, even more preferably the ratio between the length L and the largest dimension Dₘₐₓ of said first aperture 57 should be greater than 2. Most preferably the ratio between the length L and any of Dₘᵢₙ, Dₐᵥ, or Dₘₐₓ can be greater than 2.5.

Without wishing to be bound by theory it is believed that the slanted capillaries in the polymeric backsheet film of the breathable backsheet in the disposable absorbent article according to this preferred embodiment of the present invention allow air and water vapour permeability, and at the same time they can easily bend owing to the preferred L/D ratio under the pressure exerted from the wearer facing side on them by the user during the wearing time. The bending causes the slanted capillaries to close under pressure such that liquid transport through them towards the outside of the article becomes nearly impossible. Hence these three-dimensional formed film layers are highly preferable in the context of breathable absorbent articles. This also gives the polymeric backsheet film 55 a better resilience and the capability of providing a cushioning effect under the pressures in use.

As explained hereinbefore, in case of a slanted capillary having a circular first opening 57, such as that illustrated in Figure 6, no distinction between Dₘᵢₙ, Dₐᵥ, and Dₘₐₓ is necessary or possible since the three relevant dimensions D of the first opening 57 correspond to one another.

In case of slanted capillaries 54 with a first aperture 57 having a shape different from a circle the three relevant dimensions Dₘᵢₙ, Dₐᵥ, and Dₘₐₓ actually differ from one another. In order to better express the easiness of bending under an external pressure of a slanted capillary having the preferred L/D ratio, one of the three relevant dimensions of the first aperture can be more indicated, depending on the particular shape of said first opening, i.e., whether it is regular or irregular, or having a perimeter which is internally totally concave, such as for example circular, oval, or elliptic, or alternatively partially concave and partially convex. In this latter case, the use of Dₐᵥ or, more preferably, of Dₘₐₓ in the ratio L/D are preferred.

According to the present invention and as illustrated in Figures 1 and 3 to 8 the capillaries 54 of the polymeric backsheet film 55 are slanted, i.e., disposed at an angle relative to the plane P of the polymeric backsheet film which is less than 90 degrees, or, alternatively, curved or bent towards the plane of the polymeric backsheet film.

Preferably a polymeric backsheet film 55 can be made in accordance with the aforementioned US 5591510 or PCT WO 97/03818, WO 97/03795. In Figures 3 through 7 alternative embodiments of such capillaries are shown. Preferably the slanted capillaries are evenly distributed across the entire surface of the backsheet film 55, and are all identical. However, layers having only certain regions of the surface provided with apertures, for example only an area outside the region aligned with the central loading zone of the absorbent core, may be provided with capillaries according to the present invention.

Methods for making such three-dimensional polymeric films with capillary apertures are identical or similar to those found in the apertured film topsheet references, the apertured formed film references and the micro-/macroscopically expanded film references cited above. Typically a polymeric film such as a polyethylene (LDPE, LLDPE, MDPE, HDPE or laminates thereof) is heated close to its melting point and exposed through a forming screen to a suction force which pulls those areas exposed to the force into the forming apertures which are shaped such that the film is formed into that shape and, when the suction force is high enough, the film breaks at its end thereby forming an aperture through the film. Other film materials include polyesters, polyethers, polyvinyl alcohols and others.

Hydrophilic continuous films that are substantially liquid impervious, but moisture vapour permeable per se can also be used for the manufacture of the three dimensional polymeric films with capillary apertures according to the present invention. Such films do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower. These films, referred to as monolithic films, are known in the art, such as e.g. Hytrel (TM) film from DuPont, Corporation, USA.

When a monolithic film is used to manufacture a three dimensional polymeric film with capillary apertures according to the present invention, it is capable of maintaining breathability of the polymeric film with capillaries even in areas of the film where, under particularly stressed use conditions, the capillaries are totally closed due to bending, under e.g. high pressure exerted by the user.

Various forms, shapes, sizes and configurations of the slanted capillaries are possible and will be discussed in reference to Figures 4 through 7 in the following.

Figures 4 to 7 show a cross-sectional view of different embodiments of a single slanted capillary 54 of a preferred polymeric backsheet film 55 made in accordance with US 5591510 or PCT WO 97/03818, WO 97/03795, and also having the preferred ratio between the length L and the dimension D of the first opening 57, wherein elements corresponding to those already described in Figure 3 are identified by the same numerals. The centreline A forms along at least part of its length an angle of less than 90 degrees with the plane P of the film which is the same plane as the garment facing surface of the film 55. This angle should be preferably in the range between 85 and 20 degrees, more preferably between 65 degrees and 25 degrees, and most preferably between 55 and 30 degrees.

An angle of less than 90 degrees with the plane P can be formed by the centreline A along its entire length when, as illustrated in figures 4 and 5, the centreline A is substantially rectilinear or, as in figure 3, where the centreline A is rectilinear along most of its length. This angle can be said to actually corresponds to the angle of the slanted capillary.

In alternative embodiments where the centreline A is curved entirely or in part, such as for example those illustrated in figures 6 and 7, the angle of the centreline A, as it is known from geometry, corresponds to the angle of the tangent to the centreline A at a given point, as measured in the sectional view illustrated in the figures themselves. Of course in these cases the angle can vary along the length of the centreline A. It is within the scope of the present invention that the centreline A forms an angle of less than 90 degrees along at least a portion of its length, but it is preferred that substantially the whole centreline A, or at least most of it, forms a constant angle of less than 90 degrees with the plane P, as illustrated in figures 3, 4, and 5, or that alternatively the angle changes continuously along the whole length of the centreline A, as illustrated for example in figures 6 (curved capillary).

It is of course possible to allow the capillaries to take the shape of a funnel such that the second opening 58 is (substantially) smaller than the first opening 57 when considering the opening size in a plain perpendicular to the centreline A. Such an embodiment is shown in Figure 5 and Figure 4. In Figure 4 it is also shown that the wall 56 of the capillary may not end in the second opening 58 such that the opening forms a surface perpendicular to the centreline A but such that the wall on the portion of the capillary further apart from the wearer facing surface of the film 55 extends over the opening to further aid the film in reducing the probability of liquid migrating through the capillaries from the absorbent core on the wearer facing side of the film 55 to the garment facing side of the film (and cause leakage).

In Figure 6 another embodiment of the capillaries useful for the present invention is shown which is curved along its length towards the wearer facing surface of the film 55. This has a similar effect as the extension of the wall 56 as shown in Figure 3.

In Figure 7 another preferred embodiment of a capillary according to the present invention is shown which has a first portion 257 and a second portion 258. The first portion 257 of the capillary is different in direction than the second portion 258 of the capillary 54. This difference can also be in shape, size, and form of the portions of the capillary in order to achieve the desired level of breathability while preventing liquid passage through the film in a direction from the wearer facing side towards the garment facing side.

In the preferred embodiments of the present invention comprising the polymeric film backsheet 55 with slanted capillaries it is believed that said capillaries allow air and water vapour permeability which is not hindered by them being slanted at an angle or by the shape as indicated above. At the same time the slanting and shaping further help the capillaries having the preferred ratio between length L and dimension D of the first opening 57 to close under pressure exerted from the wearer facing side on them such that liquid transport through the capillaries towards the outside of the article becomes nearly impossible. Hence these three-dimensional formed film layers are highly preferable in the context of breathable absorbent articles.

A further advantage related to a polymeric three dimensional film layer having slanted capillaries and being comprised in a backsheet of a disposable absorbent article is the fact that such a three dimensional film does not allow a direct passage through its thickness, i.e., along a direction perpendicular to the overall film surface. Therefore this prevents portions of a further layer or of a material adjacent or applied to one surface of the polymeric three dimensional film with slanted capillaries from getting direct access to the opposite surface through the thickness of the film, i.e., through the slanted capillaries. This is particularly useful in the context of a breathable backsheet. The slanted capillaries in fact prevent that a bridge can be created between the two surfaces of the polymeric three dimensional film, e.g. by allowing fibres of an adjacent fibrous layer to penetrate into the capillary apertures and get in contact with a further layer adjacent to the opposite surface, so creating a preferred passage for the fluid. Further, when adhesive is used to join a polymeric three dimensional film to an adjacent layer, the slanted geometry of the capillaries does not allow that part of the adhesive can penetrate through the capillaries and emerge on the opposite surface, possibly contacting another layer and again creating a bridge trough the three dimensional film.

According to the present invention, it has been discovered that by selecting the length L of the slanted capillaries 54 as a function of the capillary geometry, namely of the size of the respective first opening 57, and also of the distance between the slanted capillary 54 and adjacent capillaries 54', it is possible to avoid that, under particularly severe wearing conditions involving higher compression and stresses, typically in a direction perpendicular to the plane P of the backsheet film 55, the second opening 58 of the capillaries 54 is brought in contact with the inner surface of the three dimensional polymeric backsheet film 55 which lies between the capillaries, which i.e. corresponds to the wearer facing surface of the backsheet film 55.

Figure 2 shows a portion of the garment facing surface of a backsheet film 55 similar to that illustrated in Figure 1, with a schematic representation of the first openings 57 of the capillaries 54, and with the central row of first openings corresponding to the row of capillaries in the sectional view of Figure 1. It has to be noted, however, that the circular shape of the first openings 57 illustrated in Figure 1 might not strictly correspond to the shape of the respective first openings 57 of the actual film 55, which could rather be more or less elliptical, with the major axes aligned with the bending direction of the slanted capillaries, depending on the geometry of the capillaries 54 and on the way the film 55 is actually manufactured. Circular first openings 57 as those represented for simplicity in Figure 2 could be seen on the garment facing surface of a backsheet film 55 having slanted capillaries 54 of a curved type similar to that shown in figure 6. This approximation is not relevant to the following considerations, however.

The term "adjacent", as used herein, is meant to indicate a capillary 54' positioned close to a capillary 54, with no other capillary interposed between them. With reference to the plan view of Figure 2 showing the garment facing surface of the backsheet film 55 with the first openings 57 of the capillaries 54 thereon, a capillary 54' is considered to be adjacent to a capillary 54, if a straight line drawn between the points P₁ in the respective first openings 57 does not cross the first opening of any other capillary in between. For example, according to this definition, the two capillaries indicated as 54' in Figure 2 are adjacent to capillary 54, while capillary 54" is not.

When considering the capillary 54, its centreline A, which, as already described, forms along substantially its entire length an angle of less than 90 degrees with the plane P, is comprised in a plane P' perpendicular to the plane P, and actually corresponding to the plane along which the sectional view of Figure 1 is taken.

The capillary 54 is separated from the adjacent capillaries 54' by a distance which is measured along the garment facing surface of the film 55 (corresponding to the plane P) between the first openings 57 of the capillary 54 and of the respective adjacent capillary 54', and corresponding to the shortest straight line drawn between the perimeters of said respective first openings 57.

When considering the adjacent capillary 54' lying along the same plane P' of the capillary 54, the distance between the respective openings 57, measured along the line of intersection of plane P with plane P', and therefore along the direction of the slanted capillary, is B, as shown in Figure 2. The first opening 57 of the capillary 54 has also a dimension D measured along the same line of intersection of plane P with plane P', and therefore passing through the point P₁ of the first opening 57. In the embodiment illustrated in Figure 2, where circular first openings 57 of the capillaries 54 are illustrated for simplicity, the dimension D actually corresponds to the diameter of the first opening 57 and of course is constant along any direction. In case of the elliptical first openings 57 which would actually correspond to the slanted capillaries 54 illustrated in Figure 1, the dimension D as defined above would rather correspond to the major axis of the ellipse.

According to the present invention, the length L of the capillary 54 as defined herein has to be longer than the sum of this distance B plus half of the dimension D of the first opening of said capillary 54 as defined above. In this case, the slanted capillary 54 has such a length, compared to both the dimension of its first opening, and to the distance from the adjacent capillary 54', that also when completely bent towards the body facing surface of the film 55, and typically along the same direction of the slanting, i.e. within the plane P', e.g. during particularly severe wearing conditions, the respective second opening 58 of the capillary 54 will not contact the body facing surface of the film 55, i.e. the bottom regions of the "valleys" of the film 55 comprised among the capillaries, where a certain amount of liquid could accumulate during the wearing time of a breathable absorbent article with a breathable backsheet comprising the backsheet film 55. The second opening of the capillary 54, upon complete bending, will in fact contact the side wall 56 of the adjacent capillary 54'.

According to an alternative preferred embodiment of the present invention the length L of the capillary 54 can be even higher as compared to what has been defined above, in order to avoid the further risk that the slanted capillary 54 may bend under compression along a direction slightly radially shifted from the plane P' which contains the centreline A of the slanted capillary 54 in its initial undeformed state.

Referring again to Figure 2, the distance between the slanted capillary 54 and the furthest adjacent capillary 54' is B', wherein said furthest adjacent capillary is the one indicated as 54' in the bottom row of Figure 2. The capillary 54 has also a dimension D' of its first opening 57, as measured along the direction of the distance B, i.e., along the line connecting the respective points P₁ of the first openings 57 of the capillary 54 and of the furthest adjacent capillary 54'. In the embodiment illustrated, this dimension D' actually corresponds to the diameter D of the circular first opening 57 of the capillary 54, but a different value would be measured in a non circular first opening, e.g. in an elliptical one as explained above.

The length L of the capillary 54 is preferably longer than the sum of said largest distance B' plus half of the dimension D'. In this case any possible contact of the second opening 58 of the slanted capillary 54 towards the body facing surface of the backsheet film 55 is prevented, also under particularly heavy compression forces that could cause the slanted capillary 54 not only to bend, but also to slightly move radially and eventually get into the "valley" between two adjacent capillaries. This alternative embodiment of the present invention is particularly preferred when the slanted capillaries 54 have the preferred ratio between their length and the respective dimension of the first opening, as disclosed herein and illustrated in Figures 3 to 7, and also described in the companion application filed at the same time as the present application and entitled "Absorbent article with improved backsheet comprising capillary apertures" (P&G Case CM2062F).

Of course the above considerations on the preferred length of the slanted capillaries and its relationship with the dimension of the respective first opening of the capillary and the distance from adjacent capillaries is valid when the slanted capillaries are arranged in a breathable backsheet film in a sufficiently closed array, where the distances between adjacent capillaries are of the same order of magnitude of the largest dimension of the first opening of the capillaries, preferably not longer than five times said larger dimension, otherwise the lengths involved for the slanted capillaries would be too big for a breathable film structure according to the present invention.

On the other hand, in alternative embodiments of the present invention the capillaries 54 of the film 55 can be arranged in such a closed array, that the perimeters of the first openings 57 of the adjacent capillaries 54 can be tangent to each other. In this case, the distance B, defined and measured as explained above, can be zero, wherein the distance B', in the same situation, is however still different from zero. The condition according to the present invention is however satisfied in a situation wherein B is zero if L is longer than D/2.

Preferred shape and size of the capillaries 54 in a three-dimensional polymeric film 55 comprised in a breathable backsheet structure of an absorbent article according to the present invention can be determined by the skilled man in order to allow a good water vapour permeability, preferably also air permeability, to the backsheet structure.

Preferably, the capillaries 54 have a first opening 57 with a dimension D generically comprised between 0.2 mm and 5 mm, wherein D comprises the smallest dimension Dₘᵢₙ, the average dimension Dₐᵥ, and the largest dimension Dₘₐₓ, as defined herein. When the first opening 57 has a circular shape, dimensions Dₘᵢₙ, Dₐᵥ, and Dₘₐₓ coincide, and correspond to said dimension D. They are preferably comprised between 0.4 mm and 2 mm, more preferably between 0.7 mm and 1.5 mm. If, as it is preferred, the first opening 57 has a substantially elliptical shape, with a Dₘᵢₙ and a Dₘₐₓ corresponding to the two axes of the ellipse, both Dₘᵢₙ and Dₘₐₓ are also more preferably comprised between 0.7 mm and 1.5 mm.

In an example according to the present invention, the three-dimensional polymeric film 55 has capillaries 54 having a funnel shape substantially corresponding to that illustrated in Fig. 4, with the centreline A forming an angle of about 35 degrees with the plane P of the film, wherein Dₘᵢₙ is 0.9 mm, Dₘₐₓ is 1.1 mm, and L is 1.9 mm.

The capillaries are arranged as illustrated in Figure 2, with the centre points P1 of the first openings 57 in triangular pitch. Same considerations already done about the shape of the first openings 57 which are actually elliptic, rather than perfectly circular as shown in Figure 2 for simplicity, also apply to this context. The major axes of the ellipses, and therefore Dmax, are aligned with plane P'. The distance B is 0.5 mm.

### Absorbent article construction

A further aspect of the present invention relates to the joining of the topsheet, backsheet and absorbent core elements to provide the absorbent article. According to the present invention at least two, preferably all of the elements of the article are joined.

Each of said elements comprising at least one layer has a wearer facing surface and a garment facing surface. Typically, adjacent garment facing surfaces form a common interface with the wearer facing surface of an adjacent element or layer. The elements or layers are joined together across this common interface. In this manner the topsheet is joined to the absorbent core, and the core is joined to the backsheet. Furthermore, each of said topsheet, backsheet and core elements may comprise more than one layer and these layers may also be similarly joined. In addition the topsheet may be directly or indirectly joined to the backsheet at the periphery of the absorbent article to contain the absorbent core.

The elements and layers thereof may be joined by any means known in the art for affixing two adjacent layers of material, such that the layers are directly attached to one another or directly attached to one another via the joining means. Suitable joining means include adhesive, fusion bonding, ultra sonic bonding, stitching, heat (e.g. thermobonding by welding fibers at intersections or melting a polymer to attach fibers or films to each other), embossing, crimping, pressure bonds, dynamic mechanical bonds or combinations thereof. According to an embodiment of the present invention the preferred means of joining is adhesive. Suitable adhesives include non pressure sensitive and cold adhesives. The adhesive may be applied by any means known in the art such as spiral application, slot coating, spraying, spiral spraying, curtain coating, contact coating and printing, provided that the adhesive does not substantially affect the breathability, in the preferred embodiment of the present invention of a breathable absorbent article, and other functions of the elements of the article.

One means of achieving this is to use particular adhesive application methods such as open adhesive application techniques, whereby areas of the common interface are adhesive free, whilst retaining the required level of attachment/joining of the two adjacent layers or elements. In particular spiral spraying is preferred.

In a preferred embodiment of the present invention wherein the absorbent article finds utility as a sanitary napkin or panty liner, the absorbent article is also provided with a panty fastening means which provides means to attach the article to an undergarment. For example the panty fastening means may comprise a mechanical fastener such as hook and loop fasteners such as marketed under the tradename VELCRO, snaps or holders. Alternatively, the article is fastened to the undergarment by means of panty fastening adhesive on the backsheet. The panty fastening adhesive provides a means for securing the article to the panty and preferably a means for securing the article when soiled, to the fold and wrap package for convenient disposal. Typically, at least a portion of the garment facing surface of the backsheet is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

In order to reduce the adverse effect on breathability of the backsheet (and thus of the article as a whole) in preferred breathable absorbent articles, the adhesive is preferably applied such that at least 60%, preferably from at least 80%, most preferably at least 90% of the surface of the backsheet is adhesive free. The required adhesiveness can still be achieved even when using reduced surface coverage by using a particular distribution such as thinner strips, discontinuous strips of adhesive, intermittent dots, random patterns or spirals.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX El/O and BL 30 MG-A SILOX 4 P/O available from Akrosil Corporation.

According to the present invention the absorbent article can be used beneficially in the context of sanitary napkins, panty liners, incontinence articles, sweatpads and diapers. However, sanitary napkins are particularly susceptible to the present invention. The disposable article may thus also have all those features and parts which are typical for products in the context of their intended use.

## Claims

1. Breathable disposable absorbent article having a garment facing surface, which is oriented to face in the direction of a garment (21) during use of the article, and a wearer facing surface, which is oriented to face in the direction of the wearer (20) during use of the article, said article comprising at least
a liquid pervious topsheet (30);
a breathable backsheet (50);
an absorbent core (40, 42, 44) comprised between said topsheet (30) and said backsheet (50);
said breathable backsheet (50) comprising at least a resilient, three dimensional web, said web consisting of a liquid impervious polymeric backsheet film (55) having a garment facing surface and a wearer facing surface, said surfaces being planar and parallel to each other, and parallel to the plane P of said backsheet film (55), said backsheet film (55) further having apertures (53), said apertures (53) forming capillaries (54), said capillaries (54) having side walls (56) which extend away from said wearer facing surface of said backsheet film (55) towards said absorbent core (40, 42, 44), said capillaries (54) having a first opening (57) in said garment facing surface of said backsheet film (55), and a second opening (58) at the end of said capillaries (54) spaced apart from said wearer facing surface of said backsheet film (55), each of said capillaries (54) defining a conduit (70), said conduit constituted by said first opening (57), said second opening (58), and said side walls (56),
each of said capillaries (54) having a centreline A defined as the geometrical axis of said conduit (70), and a length L measured along said centreline A between two points P₁ and P₂ respectively corresponding to said first opening (57) and said second opening (58) as described herein,
said centreline A forming along at least part of its length L an angle of less than 90 degrees measured from said plane P,
said centreline A being comprised in a plane P' perpendicular to said plane P of said backsheet film (55),
each one of said capillaries (54) being separated from adjacent capillaries (54') by distances measured along said garment facing surface of said backsheet film (55) between respective first openings (57) of said capillary (54), and of said adjacent capillaries (54')
wherein the distance measured along the line of intersection of said plane P with said plane P' is B,
and wherein said first opening (57) has a dimension D measured along the direction of said distance B and passing through said point P₁,
said article being characterised in that
said length L is longer than the sum of said distance B plus half of said dimension D.

2. Breathable absorbent article according to claim 1, characterized in that said length L is longer than the sum of a distance B', plus half of a dimension D',
wherein said distance B' is the largest distance measured between respective first openings (57) of said capillary (54) and of an adjacent capillary (54') as defined herein,
and said dimension D' is a dimension of said first opening (57) of said capillary (54) as measured along the direction of said distance B' and passing through said point P₁.

3. Breathable disposable absorbent article according to claim 1, characterized in that said first opening (57) has a smallest dimension Dₘᵢₙ, an average dimension Dₐᵥ, and a largest dimension Dₘₐₓ as defined herein, wherein the ratio between said length L and said smallest dimension Dₘᵢₙ is larger than 2, preferably wherein the ratio between said length L and said average dimension Dₐᵥ is larger than 2, more preferably wherein the ratio between said length L and said largest dimension Dₘₐₓ is larger than 2.

4. Breathable absorbent article according to claim 3, characterized in that said ratio is larger than 2.5.

5. Breathable disposable absorbent article according to any of the preceding claims characterised in that at least some of said capillaries form cylinders having liquid transport areas which are constant in a direction towards the absorbent core (40, 42, 44) when comparing areas perpendicular to said centreline A.
